(19) Europäisches Patentamt
European Patent Office
Office européen des brevets

(11) **EP 3 579 916 B1**

(12) **EUROPEAN PATENT SPECIFICATION**

(45) Date of publication and mention
of the grant of the patent:
**20.10.2021 Bulletin 2021/42**

(51) Int Cl.:
*A61N 1/30* *(2006.01)*    *A61N 1/04* *(2006.01)*
*A61N 1/32* *(2006.01)*    *A61N 2/02* *(2006.01)*
*A45D 34/04* *(2006.01)*    *A61H 15/02* *(2006.01)*
*A61H 23/02* *(2006.01)*    *A61M 35/00* *(2006.01)*
*A61M 37/00* *(2006.01)*

(21) Application number: **18720389.8**

(22) Date of filing: **08.02.2018**

(86) International application number:
**PCT/IT2018/050016**

(87) International publication number:
**WO 2018/146709 (16.08.2018 Gazette 2018/33)**

(54) **DEVICE FOR INTRACELLULAR TRANSPORT IN RESONANCE**

VORRICHTUNG ZUM INTRAZELLULÄREN TRANSPORT IN RESONANZ

DISPOSITIF DE TRANSPORT INTRACELLULAIRE EN RÉSONANCE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.02.2017 IT 201700014315**

(43) Date of publication of application:
**18.12.2019 Bulletin 2019/51**

(60) Divisional application:
**20204616.5 / 3 824 947**

(73) Proprietor: **Cetroni, Bruno Massimo
23025 Novate Mezzola (Sondrio) (IT)**

(72) Inventor: **Cetroni, Bruno Massimo
23025 Novate Mezzola (Sondrio) (IT)**

(74) Representative: **Sach, Greg Robert et al
STERAF S.r.l.
Corso di Porta Romana 132
20122 Milan (IT)**

(56) References cited:
**EP-A1- 1 178 854      WO-A1-2013/190489
US-A- 5 421 817      US-A- 5 833 647
US-A1- 2002 010 414      US-A1- 2002 038 101
US-A1- 2002 065 533      US-A1- 2003 233 085
US-A1- 2005 065 461      US-A1- 2011 098 632
US-A1- 2012 004 591      US-A1- 2014 114 234
US-A1- 2014 187 851      US-A1- 2015 018 752**

**Description**

FIELD OF THE INVENTION

This invention relates to a device for resonance intracellular transport.

BACKGROUND STATE OF THE ART

In brief, it should be noted that hydroelectrophoresis, developed and implemented since 1989, is an evolution of the classic iontophoresis therapy.

Hydroelectrophoresis was presented to the medical field with a better passage of ions and also with a passage of small molecules through the skin, by means of new bipolar waves, which improved transdermal transmission with respect to classic iontophoresis, by virtue of acquisition of these modified bipolar currents.

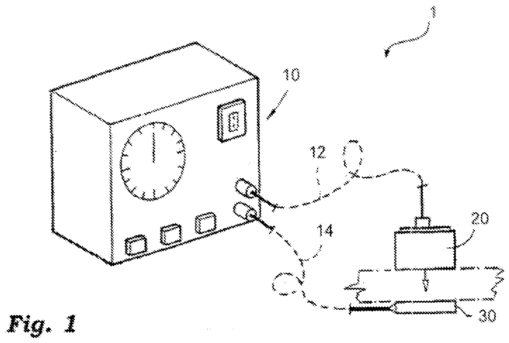

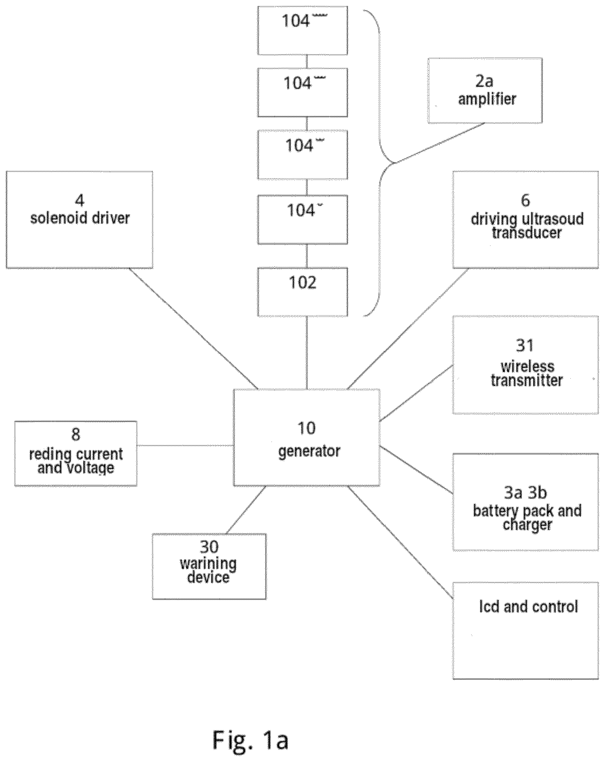

Figure 1 shows an example of a device for transdermal transport overall designated as 1.

In the example considered, device 1 comprises a wave generator 10 to which two electrodes 20 and 30 are connected by means of respective cables 12 and 14.

In particular, in the example considered, electrode 30 is made of a conductive material and can be of any shape, though a flexible metal lamina is preferable. Said flexible metal lamina can in fact be applied adapting itself to the anatomical shape of the body area of the patient on which this electrode is to be applied.

In the embodiment considered, the electrode 20 is a handpiece made in such a way as to contain an active principle to be administered by the transdermal route. For example, the active principle may be contained in a liquid or frozen solution, a gel or other. In general, the term "active principle" may include a drug, a product to treat skin blemishes, such as cellulitis or similar, or ions and/or molecules in general.

For example, Italian patent application FI99A000141 describes a wave generator that generates a special waveform that can be used for hydroelectrophoresis.

Italian patent applications FI98A000137 and FI99A000055, on the other hand, describe possible embodiments of the handpiece 20.

The particular waveform used, together with the gel formulation, allow the drug to penetrate the tissues as deeply as up to approximately 11 cm. This depth is greater than can normally be achieved with other electrophoretic methods (by just a few mm). Moreover, high local concentrations of the drug used can be achieved.

Another known document WO 00/74774 describes various embodiments, the device for transdermal transport can be connected to two electrodes, wherein one electrode is configured to contain an active principle to be administered by the transdermal and/or intradermal route. The device also comprises a wave generator, which generates a driving signal for the electrodes.

The driving signal includes a plurality of packets grouped in trains of packets and in groups of trains. In particular, each packet consists of a unidirectional signal resulting from the combination of a modulating signal, for example with a frequency of between 0.1 and 5 Hz, and a carrier signal, with a frequency of between 200 and 2000 Hz, for example. Moreover, each train of packets consists of a series of packets and each group of trains comprises a series of trains of packets. Specifically, in various embodiments, the trains of packets comprise packets with the same polarity and same duration, but at least a first packet has a carrier signal with a first frequency and at last a second packet has a carrier signal with a second frequency.

The wave generator reverses the polarity of the trains of packets after a given time interval. For example, in various embodiments, the wave generator reverses the polarity of each train of packets with respect to the polarity of the preceding train of packets.

Document WO 2013/190489 describes a device for transdermal and/or intradermal transport. The device comprises a wave generator configured for generating a driving signal to be sent to the electrodes. In one embodiment, the driving signal comprises a plurality of packets grouped in trains of packets and in groups of trains, wherein each packet consists of a unidirectional signal that results from the combination of a modulating signal and a carrier signal, wherein each train of packets consists of a series of packets, and wherein each group of trains comprises a series of trains of packets. The waveform produced allows greater tissue penetration by intradermal or transdermal transport.

Document US 2003/0233085 discloses a method of enhancing the permeability of the skin to a biologically active permeant or compound. The method utilizes a combination of sonophoresis and chemical enhancers. The previous preparation of the skin using an ultrasonically generated mechanical skin scrubbing action is also described. Synergism brought simultaneously applying iontophoresis, electroporation, mechanical vibrations and magnetophoresis is used to optimize the transcutaneous active permeation of compounds, considerably lowering the time of treatment.

Document US 2011/0098632 discloses a device for ocular application of an active principle comprising a main electrode including an insulating layer, an adhesive layer designed to bind the insulating layer to a conductive layer, characterized in that the main electrode comprises a zone designed to be urged into contact with an eyelid.

Document US 2014/0114234 discloses a device for transmitting acoustical energy through the surface of the skin of a patient, coupled with the administration of a compound, for example an acne medication, for the purpose of temporarily expanding the patient's pare size and enhancing the delivery of the compound to the patient's pores. Tue ultrasonic transducer emits a sonic transmission of variable intensity and frequency for the purpose of enhancing the absorption of compounds that have first been deposited on the surface of the skin and into skin pores that have been expanded by the ultrasound transmission.

One of the objects of this invention is to increase the therapeutic effect achieved with the known devices, by increasing the local and not systemic action of the molecules transported through the cell membrane.

This invention also aims to succeed in causing the molecules transported through the cell membrane to activate the cell's metabolism.

Essentially, an object of this invention is to describe a device that will stimulate resonance transport, surpassing the normal transdermal and/or intradermal techniques, thereby essentially offering an advantageous method in terms of a plurality of innovative aspects.

Finally, this invention aims to increase local therapeutic action.

BRIEF SUMMARY OF THE INVENTION

These objects and others which shall become clear in the course of the description are achieved by means of an innovative device for the resonance transport that will be described hereunder.

The invention is as defined in the appended claims. Aspects, examples and embodiments of the present disclosure which do not fall under the scope of the claims, in particular methods, do not form part of the invention and are presented for illustration purposes only.

It is considered very important in this case to provide an accurate definition of what the applicant means by "resonance": the term normally used would be "bioresonance", but that term has been used incorrectly over time. Therefore, the applicant hereby specifies that resonance in this case reverts to the technical definition provided below, which will be further supported by the bibliography annexed hereto. In fact, the "cellular resonance" technique, which is of interest here, is widely documented in science.

As such, resonance is to be understood herein as electromagnetic resonance, in other words emission, by means of an artificially generated electromagnetic field, of electromagnetic waves (EMW) that resonate with the same waves produced by the cells of the human body. By way of an illustrative example, think of a normal radio signal that the cell is able to decipher to respond to it adequately, thanks to the coherent nature of the signals.

In this way it is therefore possible to communicate with a body by giving information that is coherent with the normal functioning of the body: as though the body were a mobile telephone that rings when its coherent signal arrives, and the voice - i.e. other waves - is transferred on that wave. (See: M. Covelli ATTI XVI SNAMID National Congress, February 2003: Frohlich H. Biological coherence and response to external stimuli. Berlin, Heiderberg, Sprinder, 1988; A.R. Liboff "Cyclotron resonance in membrane transport" in A. Chiabrera et al. (Eds) Interactions between electromagnetic fields and cells, pp. 281-296, Plenum Press, 1985; many others in the bibliography provided)

Quotation: "It should be underlined that the molecular aggregation of living biological tissue occurs exclusively within a water molecule. Therefore, water is both a solvent for the molecules of the biological tissue and an environment in which they develop. Water clusters can be affected and modified by the coherent electromagnetic fields. (reference Masaru Emoto, Giuliano Preparata, Ricciardi, Bodo Kohler, Arcieri)." If we consider DNA and its classic double helical representation, we will notice that DNA, too, (Garyaev) has a resonance frequency of its own, which corresponds to the cellular resonance frequency. End of quotation -

The intensities of the EM field are therefore very low (in the region of a few to a few hundred photons per second per square centimetre of emission surface). One of the sources is DNA; ultrafine or Threshold electromagnetic fields transmit essential information, such as the regulation of the biochemical reactivity of the membrane potentials (*i.e.* active transport of substances inside and outside the cell, nutrients from one side, waste from the other), transmission of nerve impulses (i.e. the electrical command signals, for example to the muscles, also to the heart), immunostimulation, growth regulation, biological rhythms, etc...". Bases of reference: F. A. Popp, "Recent Advances in biophoton research and its application", World Scientific, 1992; Blank Martin, Goodman Reba, "Do electromagnetic fields interact directly with DNA?" Bioelectromagnetics, 18:111 5, 1997; Gariaev P.P. Wave genetic code. Moscow, Izdatcenter, 1997; Gariaev P.P. Genetic structures as source and receiver of holographic information. Together with I.V. Prangishvili, G.G. Tertinshny and others.//Sensors and systems, 2000. No. 2(11).P2_8. And others in the bibliography).

A classic example of cellular resonance frequency used in medicine is NMR (nuclear magnetic resonance).

In particular, for the requirements of this invention, the principle of operation is the same: the human body is two-thirds water, and the quantity of water varies according to the tissues and any pathological conditions they may have. A strong external magnetic field makes the body's water molecules change energy state and excites the protons. When a radio wave of suitable frequency to resonate with the protons is passed, their energy changes and they return to their original conditions. The oscillations induced in the protons can be detected from externally by measuring the density of molecules in that point. When processed by a computer, this information will, finally, give an image of the organs explored by the machine; if tissues contain a lot of water, they appear very light, whereas if they contain little water, they are dark. With their work in the 1970s, the two Nobel Prize winners (Felix Bloch and Edward Purcell), one a chemist and the other a physicist, lay the foundations for application of this powerful examination instrument in humans (Raymond Vahan Damadian/ Paul Lauterbur biological effects of magnetic fields).

The subject matter of this invention is therefore an innovative device for resonance intracellular transport.

The advantages and objects of this invention shall be achieved thanks to the innovative device as defined in the appended claims.

Therefore, the at least one frequency/carrier signal is suitable for modulating a pulse that determines the depth of transfer of the active principle and the at least second frequency/modulating signal groups and modulates said pulse into at least four packets having the same pulse frequency.

The further modulated modulating signal modulates and groups the packets into trains, which can have the same or different pulse frequencies within a group of trains and the modulating signal groups and modulates the trains into groups of trains, and the modulating signal modulates and groups the groups of trains into groups of groups of trains and reverses the polarity of the group of trains with respect to the preceding group of trains, the latter preferably occurring every 2 minutes

This is in order to obtain the emission of the full spectrum of resonance frequencies of the area to be treated, these frequencies being univocally defined, as stated above and as shall be described hereunder, to act exclusively on the chosen area, therefore in a totally innovative and advantageous way, without dispersions in circulation.

In particular, said solenoid is suitable for generating an electromagnetic field able to make the water clusters coherent with the currents generated by electrophoresis.

Furthermore, the ultrasound generator breaks the bonds of the stratum corneum, thus creating more space between corneocytes (the cellular component of the stratum corneum), distancing them from one another, all this enabling more effective action.

Therefore, said wave generator or signal, already present in the known art, is advantageously innovatively modified here to produce waves of various forms, including sine, triangle and square waves, etc. with frequency of 0 to 2200 Hz and variable currents and voltages, and is built to generate several carrier signals at the same time, each carrier signal further characterised in that it modulates and groups the signals, or pulses, into packets, the packets into wave-trains, the wave trains into groups of trains, the groups of trains into further groups of trains, and is also made to be able to reverse the polarity of one or more signals.

An advantage of this solution is that the particular combination of trains of packets can be modulated so as to match the specific organ of the human body to be treated, on each occasion, reaching a given area with precision.

In particular, with said device combined with a handpiece which will be described hereunder, forming an adequate system, desired uniform transport is obtained, through the extremely accurate control of transport times at the selected depths. Consequently, the treatment guarantees better results overall, as the tissues are more inundated with molecules in a uniform way, thus preventing the dispersion of the transported products within the body.

Moreover, even more advantageously, application and transport times are reduced.

Furthermore, thanks to the solution described above, the device is able to reach depths of up to 10 cm and thicknesses of up to 3 cm.

In general, the depth and thickness to which molecules can be transported is determined by the frequency of the pulses described above, depending on the waveform and its envelope.

In particular, in this case, transport occurs via the water channel, unlike in previous methods that exploited the ion channel. When the ion channel is used, the molecules of the active principle encounter the cell's ion filters, which adversely causes reduced penetration and dispersion of the active principles in circulation. The result obtained was inferior and potentially caused an immune system response in the case of allergies.

The innovative device and method described herein, which instead takes advantage of the water channel on the outside of the cell membrane, significantly increases the amount of active principle actually reaching the area to be treated, with almost no (less than 5%) dispersion of the active principle in circulation. An exclusively localised, and not systemic, effect can be established.

In fact, no resonance modulating frequencies were present in the previous methods (repetition frequencies of the trains and repetition frequencies of the packets were not associated with a particular systemic effect or desired result, but were simply convenient, or arbitrary, choices); what is more, they provided for at least one pulse frequency within a train being different from the others, which compromised penetration and uniform transfer.

Ultimately, with reference to the abovementioned document WO2013/190489, which describes a previous method described by the same known applicant, which in that case provided for transdermal transport (a method that we aim to surpass with the resonance method described by this invention), which occurred according to modulation of the repetition frequencies of the packets and of the trains, those frequencies, unlike in the method described by this invention, were arbitrary. Another significant factor of the previous invention was the use of the ion channel to transport substances, whereas this invention uses the water channel, made possible also by the coherence of the agarose gel obtained thanks to the solenoid driven by the device and present on the handpiece. In fact, the previous invention had a transdermal transport method with significant dispersion of the transported active principles in circulation, for the reasons stated above.

That same known method expressly required that, within a train of packets, at least one packet had a different pulse frequency from the others: this type of specific implementation does not allow constant migration or uniform transfer of product in the area to be treated, due to the too short duration of the signal, for each individual depth frequency: if the second packet in a train has a different depth frequency to the first packet, the duration of the signal bringing out the migration of the first packet is too short; instead, by keeping the depth frequency within the train constant, the new device/method produces a longer duration for the depth of migration.

Technical studies have demonstrated that unparalleled results can be obtained in terms of the quantity of active principle transported and the transport time of the same, by keeping a minimum of four packets with the same frequency within a given train.

The known resonance frequencies set out below in table 1 are instead advantageously exploited in the invention described herein.

In this invention, on the other hand, the use of the resonance frequencies as described advantageously serves to stimulate the response of the relative cell receptors exclusively in the area to be treated, by applying the method of repetition of trains, packets, etc. described herein in detail. This makes it possible to essentially prevent dispersion in circulation, limiting it to a maximum of 5%.

Secondly, thanks to the coherence of the clusters in the solvent (agarose gel) thanks to the abovementioned solenoid, molecular transfer capacity has increased, resulting in faster treatment. The previous methods took 45 minutes to transport 30 ml of saline gel with a concentration of 2 grams of active principles, via the transdermal route, and 25% of that gel remained outside the dermis. In this case, this method is advantageously able to transfer 60 ml of agarose gel, with an active principle concentration of up to 15 grams, in a maximum of 24 minutes (with 5% remaining on the dermis). In particular, the ultrasound emitter envisages a non-linear sweep in emission frequency of between 20 and 40 kHz inclusive.

This particular frequency, or a non-linear sweep between 22.5 and 40 kHz, acts exclusively on the stratum corneum. More specifically, said frequency acts by breaking the bonds of the stratum corneum, thus creating more space between the corneocytes, distancing them from each other, all this enabling more effective action by the dispenser roller, as better explained below.

According to another aspect of this description, the active principle is dissolved, as mentioned previously, in agarose-based gel.

More specifically, the solution used contains agarose with the possible addition of one or more other carrier substances.

The use of a saccharine glycoside gel, unlike in the known transdermal transport technique in which the gel has to be saline (as also in the aforementioned WO document), is a highly important aspect to transport substances for cosmetic and pharmacological use, as cells prefer feeding on sugar, so in the days following application, the cells of the treated body continue feeding and working, thus obtaining a long-lasting result.

Therefore, the gel is made up of water, in which agarose is dissolved, wherein the clusters (Definition of cluster: molecules of water that are held together by a hydrogen bond due to the attraction between the positive nucleus of the hydrogen and the negative atom of the oxygen) are made coherent with the currents delivered by electrophoresis and with the resonance frequencies of the tissue/organ to be treated, by an electromagnetic field generated by a solenoid (described hereunder) appropriately driven with a programmed device.

It should be noted that no device/method makes water clusters coherent for trans/intradermal transport; the latter is made possible thanks to what is described above in relation to the coherence of clusters, obtained thanks to the innovative device.

For non-ionisable or heavy substances (as in the case of chemotherapeutics), on the other hand, it is preferable to add an additional carrier to the agarose used to break down the stratum corneum barrier. Returning to the device, according to an aspect of this description, the first electrode comprises an ultrasound acoustic generator, wherein said acoustic generator is driven by means of said driving signal.

Moreover, the device can have current or voltage control to keep the amplitude of the signal constant, given that different areas of the body can have different impedance during treatment. Consequently the device self-regulates to keep the flow of molecules transferred constant and at the same time to prevent any electric shocks due to a sudden change in the conditions.

According to a further aspect of this description, the handpiece used with the device comprises a surface provided with knurling or grooves suitable to create microchannels in the stratum corneum of the epidermis.

Advantageously, said roller, thanks to the opportunely created grooves, produces microchannels that enable the gel to come into direct contact with the dermis by passing through the stratum corneum, thus cancelling out any resistance.

Even more advantageously, said handpiece has a "spray head" shape, so the gel coming out of the handpiece can be dosed in the quantity desired and at precise points; the shape of the spreading surface makes it possible to stop as long as desired or necessary on a given area, thus obtaining greater product transport precision in the areas where it is necessary.

Lastly, the handpiece comprises a dispenser with an essentially plane or convex surface, from which the said roller protrudes, where said plane or convex surface (or in any case of another shape suitable for the purpose) of the dispenser has an area of at least 5 cm$^2$, meaning the roller can stay still for a sufficient amount of time in the point where the product needs to be transported, which makes it different from commonly used handpieces. Also, this innovative surface also makes it possible to act in the corners of the handpiece, thus making it possible to reach body parts that were previously hard to access.

Moreover, in an innovative manner, it is possible to insert a solenoid into said handpiece, thanks to the shape of the latter, in order to direct the magnetic field in the handpiece all around in a consistent way.

Further characteristics and advantages of the invention can be inferred from the dependent claims.

BRIEF DESCRIPTION OF THE FIGURES

Further characteristics and advantages shall become evident by reading the following description provided by way of a non-limiting example, with the help of the figures shown in the tables annexed hereto, wherein:

- Figure 1 shows a generic device for resonance transport;
- Figure 1a shows a block diagram of a preferred embodiment of the device for resonance transport;
- Figures 2 to 9a show details of a signal useful for resonance intracellular transport according to this description;
- Figure 10 shows a handpiece for a device for resonance intracellular transport according to an aspect of the invention.

DETAILED DESCRIPTION OF SOME WAYS TO CARRY OUT THIS INVENTION

The following description illustrates various specific details aimed at allowing in-depth understanding of the embodiments. The embodiments may be carried out without one or more of the specific details, or with other methods, components, materials, etc. In other cases, known structures, materials or operations are not shown or described in detail, in order to prevent making various aspects of the embodiments unclear.

Reference to "an embodiment" within the context of this description means that a particular configuration, structure or characteristic described in relation to the embodiment is included in at least one embodiment.

Therefore, phrases such as "in an embodiment", which may be present in different places in this description, do not necessarily refer to the same embodiment. Furthermore, particular conformations, structures or characteristics may be appropriately combined in one or more embodiments.

The references used herein are for convenience purposes only and do not therefore define the scope of protection or the scale of the embodiments.

To facilitate a better understanding of the invention, some information shall be provided here, which is however known by technicians operating in the field, on the structure of the stratum corneum of the epidermis, which is made up of both the cellular component (i.e. corneocytes), and the space existing between them, which is rich in lipids with very important functions. The majority of these are: ceramides (which connect together corneocytes), cholesterol and free fatty acids that bind to both the corneocytes in the outermost layers and to those deeper down.

The stratum corneum can be informally compared to a brick wall, in which the stones represent the corneocytes and the mortar, the lipid-rich space between the cells.

Corneocytes are large flat cells which become bigger with age, as the epidermis is renewed more slowly and they remain longer in the area nearer the surface. During their migration to the outer layers, they take on keratin, bit by bit, and become corneocytes, forming the stratum corneum. Differentiation concludes with desquamation to make way for new cells.

The entire stratum corneum can be seen as the skin's external barrier: just one corneocyte protects between 1 and 20 basal cells under its area.

The outermost layers also contain large quantities of lipids produced by the sebaceous glands (triglycerides, wax esters and squalene).

To improve the connection between the cells and prevent them from sliding on top of each other, the corneocytes are secured to each other by protuberances called desmosomes.

The water content of the corneocytes is essential and is affected by ambient temperature and humidity level. The cells tend to become dehydrated if the surrounding air is very dry; if it has high moisture content, they can absorb a lot of water. In any case, the water cannot penetrate the stratum corneum in high quantities, due to the presence of the lipids between the cells. The integrity of the barrier is crucial for the skin's selective permeability. Some conditions such as psoriasis and atopic dermatitis destroy said barrier.

In general, even though a combination of all three mechanisms produces the best result, the mechanisms described above may also be used individually.

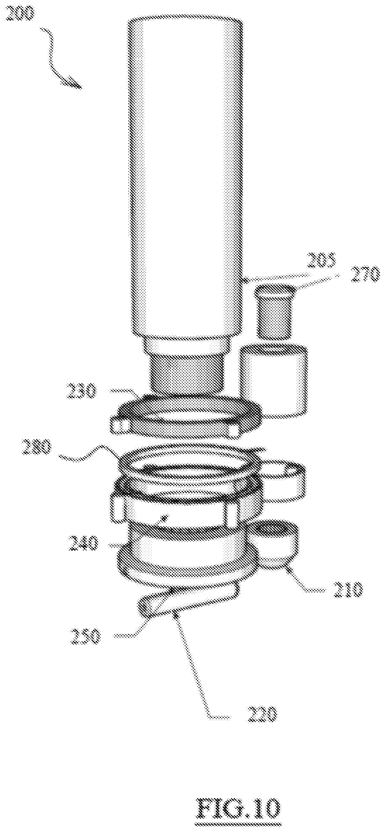

As mentioned previously, the invented device uses the following signal generated by a wave generator 10 and applied by means of connection cables 12 and 14 to two electrodes 20 and 30, wherein one of the two electrodes is designed to contain a carrier containing the active principle, all of which is better described below with reference to Figure 10.

Figure 1a shows an example, in schematic form, of a particularly preferred embodiment of the device for resonance cellular transport innovatively described herein.

In particular, said device comprises at least one wave generator 10' or signals already present in the known art, but in this embodiment, it has been innovatively created to produce waves of various forms, including sine, triangle, square waves, etc. with frequency of 0 to 2200 Hz and variable currents and voltages, and built to generate a number of carrier signals at the same time, each carrier signal further characterised in that it modulates and groups the signals, or pulses P, into packets, the packets into wave-trains Tr, the wave trains into groups of trains Tg, the groups of trains into further groups of trains, and also made to be able to reverse the polarity of one or more signals, by means of specific device programming. Furthermore, the device comprises a feeder 2 and signal amplifier 2a, designed to amplify the signal generated by generator 10' comprising also a condenser discharge system in order to prevent electric shock during the change in polarity of the signal; there is also a battery pack and corresponding charger 3a and 3b, and also an output stage 4 to drive a solenoid on the handpiece and relative amplification (the signal is picked up by the generator 10' and then amplified and regulated according to the solenoid that will be connected); an audible warning device 30 is also included, which is actuated by a square wave generated by the generator 10' when there is a change in polarity of the signal.

The device also comprises an ultrasound transducer driving stage 6 which will act as a function generator for a signal of between 20 kHz and 40 kHz inclusive, preferably 22.5 Kz and performs a non-linear frequency sweep from 22.5 to 40kz.

Furthermore, in an innovative manner, a circuit 8 is present, dedicated to reading the currents and voltages emitted: said circuit makes it possible to control the actual emission of the currents and enables the system to self-regulate its voltage and/or current depending on the changes in impedance in the human body. Impedance is generated by the body positioned between the two electrodes (one electrode is positioned in the handpiece - the spray head- and the other is the second electrode); the measurement is carried out by a dedicated feedback circuit in the device. Once the intensity has been set according to the patient's level of tolerance, the device shall keep said intensity (under current or voltage) constant, despite the changing impedance of the part. Circuit 8 sends the reading to stage 1 of the device and the latter automatically regulates the intensities in current or voltage.

This ensures uniform transport of molecules in the whole area to be treated and prevents any electric shocks in case of significant changes in impedance.

Lastly, the device comprises a wireless transmitter 31, for example WiFi and a 3G/4G connection module, GPRS UMTS, etc. able to connect the device itself to one or more networks to allow it to connect remotely to one or more data exchange terminals (e.g. for device recognition, gel consumption, etc.).

The signal driver typically has a current output, for example with maximum intensity of approximately 15-100 mA. However, the driver could also have a voltage output.

In various embodiments, the signal comprises a carrier signal with frequency of between 200 and 2000 Hz modulated in amplitude by means of a modulating signal with a frequency of between 0.1 and 5 Hz, preferably between 0.5 and 2 Hz.

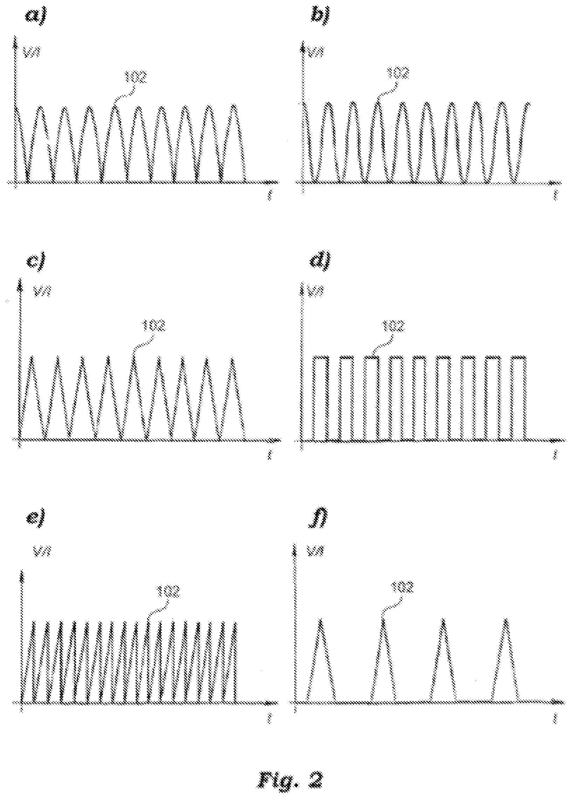

For example, Figures 2a to 2f show examples of carrier signals 102, composed, in order, of: a rectified sinusoidal waveform, a positive sinusoidal waveform, a triangular waveform, a positive square waveform, a sawtooth wave and a series of pulses spaced at intervals apart. However, in general, the carrier signal has a waveform oscillating periodically between zero and a maximum amplitude value.

For example, in one embodiment, the carrier signal is a positive sine wave whose amplitude as a function of time t has the following equation:

$$f(t) = \sin(2\pi \cdot f_p \cdot t) + 1 \quad (1)$$

where $f_p$ is the frequency of the carrier signal.

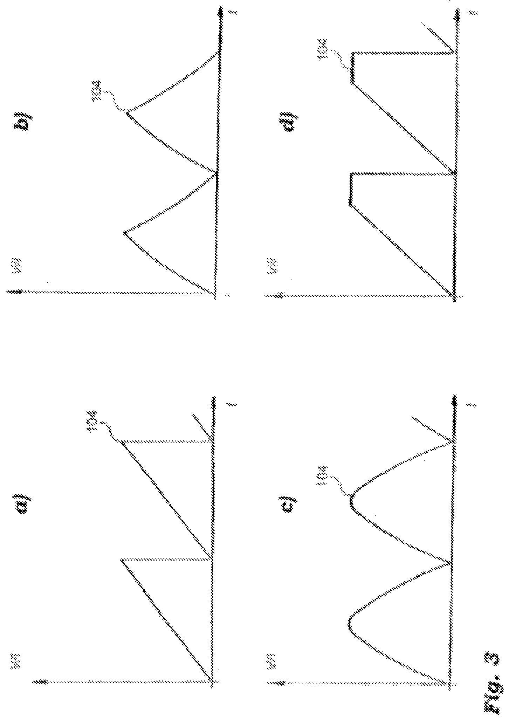

The modulating signal can also have different waveforms. For instance, Figures 3a to 3d show examples of modulating signals 104 composed, in order, of: a sawtooth waveform, a triangular waveform, a rectified sinusoidal waveform and a trapezoidal waveform. Therefore, in general, the modulating signal 104 also has a waveform oscillating periodically between zero and a maximum amplitude value.

For example, in one embodiment, the modulating signal 104 is a rectified sinusoidal wave, whose amplitude as a function of time t is given by:

$$f(t) = \left| \sin(2\pi \cdot f_m \cdot t) \right| \quad (2)$$

where $f_m$ is the frequency of the modulating signal. For instance, in various embodiments, the modulating signal has a frequency $f_m$ of between 0.1 and 5 Hz, preferably substantially equal to 0.5, 1 or 2 Hz.

Therefore, according to this description, a packet is created with a duration $T_{pac}$ corresponding to the duration of a period $T_m$ of the modulating signal:

$$T_{pac} = T_m \quad (3)$$

For instance, in various embodiments, the period of the modulating signal $T_m$ is between 0.3 and 0.8 s, preferably between 0.4 and 0.6 s, preferably 0.5 s. For example, for a modulating signal with a rectified sinusoidal waveform, the period of the modulating signal $T_m$ would correspond to $1/(2f_m)$, i.e. only to the period of a half-wave. For instance, if the period of the modulating signal $T_m$ is equal to 0.5 s, then the rectified sine wave of the modulating signal 104 would have a frequency of 1 Hz.

For example, for the carrier signal according to equation (1) and the modulating signal according to equation (2), the packet would have the following waveform:

$$f(t) = \left| \sin(2\pi \cdot f_m \cdot t) \right| \cdot \left( \sin(2\pi \cdot f_p \cdot t) + 1 \right) \quad (4)$$

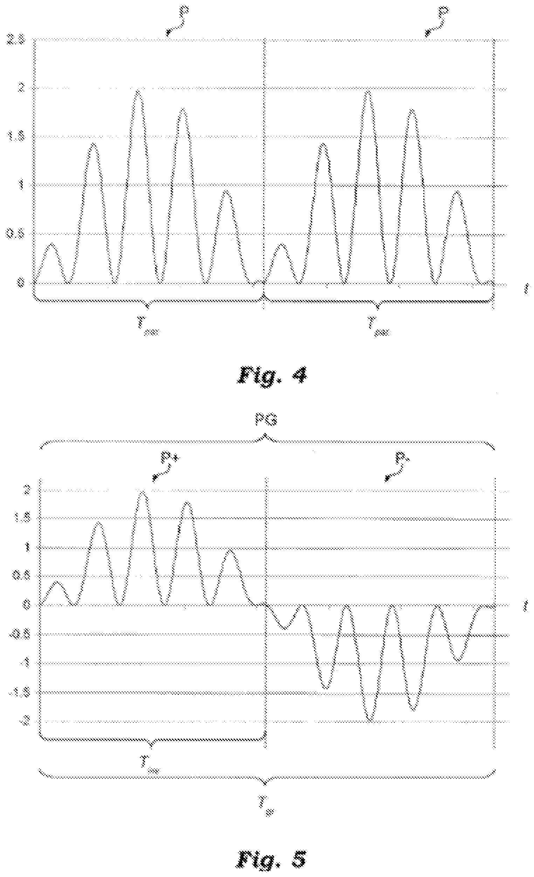

For instance, Figure 4 shows a possible embodiment of a signal comprising a sequence of two packets P.

In various embodiments, the polarity of these packets, which by definition are unidirectional, is periodically reversed. For example, in various embodiments, said polarity reversal is carried out after a time $T_{inv}$ which corresponds to the time of a group of trains, or a time that corresponds to a multiple of the time of a group of trains: for example, approximately 2 minutes

$$T_{inv} = i \cdot T_{pac} = i \cdot T_m \quad (5)$$

where i is an integer greater than zero.

In particular, in various embodiments, groups of packets are formed, comprising a number of packets with a first polarity followed by the same number of packets i with reversed polarity, i.e. the duration $T_{gr}$ of a group of packets is:

$$T_{gr} = 2 \cdot T_{inv} = 2 \cdot i \cdot T_m \quad (6)$$

In various embodiments, the frequency $f_p$ of the carrier signal remains constant for a group of packets.

For example, Figure 5 shows a possible embodiment of a group of packets PG, comprising a packet with positive polarity P+ followed by a packet with negative polarity P-.

Therefore, in the embodiment considered, the polarity is reversed after each half-wave of the modulating signal 104, which means that modulating signal 104 behaves like a normal sine signal, not a rectified one:

$$f(t) = \sin(2\pi \cdot f_m \cdot t) \cdot \left( \sin(2\pi \cdot f_p \cdot t) + 1 \right) \quad (7)$$

Moreover, the inventors observed that the penetration depth of the active principle depends mainly on the frequency of the carrier signal. Specifically, the penetration depth p can be approximated using the following equation:

$$p = \frac{(2000 \text{ Hz} - f_p)}{180} \text{ cm} \quad (8)$$

i.e. the frequency $f_p$ of the carrier signal can be calculated according to the penetration depth p required:

$$f_p = \left( 2000 - \frac{180 \cdot p}{cm} \right) Hz \quad (9)$$

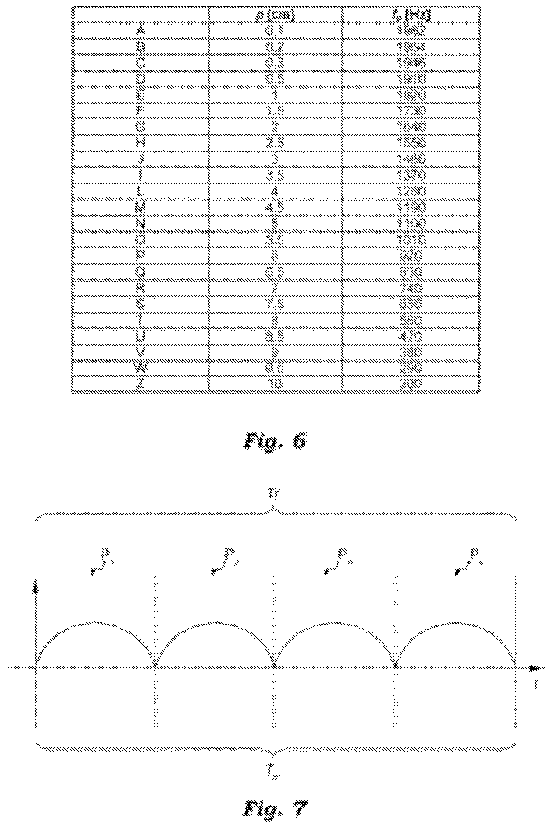

For instance, Figure 6 shows a table with 23 typical depths, identified using the letters from A to Z, with the corresponding penetration depth p measured in cm and the respective frequency $f_p$ of the carrier signal measured in Hz.

In particular, the inventors observed that treatment efficiency can be improved, by creating a sequence of signals with different carrier frequencies $f_p$.

For example, in various embodiments, a train of packets $T_r$ is created, comprising a sequence of a plurality of packets P, wherein the frequency $f_p$ of the carrier signal of each packet P is reduced, thereby encouraging the active principle to move downwards in depth.

For instance, Figure 7 shows an embodiment of a train of packets $T_r$ comprising four packets $P_1$, P2, $P_3$, and P4.

These trains of packets are periodically repeated in various embodiments.

Moreover, in various embodiments, the frequency $f_m$ of the modulating signal remains constant for the entire train of packets. Therefore, in the embodiment considered, the duration of a train of packets $T_{tr}$ is:

$$T_{tr} = 4 \cdot T_{pac} \quad (10)$$

For example, if the packet has a duration $T_{pac}$ of 0.5 s, the train would have a duration $T_{tr}$ of 2 s.

Therefore, in the currently preferred embodiment, the wave generator 10 is configured to generate a signal that comprises trains of packets Tr, wherein each train of packets Tr comprises a plurality of packets P. In particular, the packets P consist of a unidirectional signal resulting from the combination of a modulating signal 104 and a carrier signal 102. Moreover, while the frequency $f_p$ of the carrier signal remains constant for a packet P, the frequencies of the carrier signals $f_p$ of the packets P within a train of packets Tr are all the same as each other.

As mentioned previously, in the currently preferred embodiment, the train of packets Tr comprises four packets P.

In various embodiments, these trains of packets Tr are used to form groups of trains.

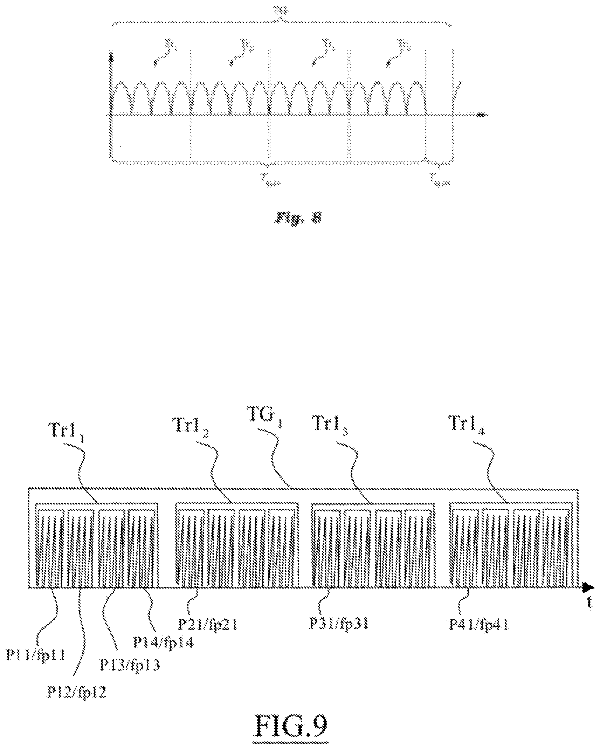

For instance, Figure 8 shows a possible embodiment of a group of trains TG.

In particular, in the embodiment considered, each group of trains TR comprises a plurality of trains Tr followed by a pause $T_{tg\_off}$, preferably lasting between 0.1 and 5s, in which the signal is constant, for example at zero. Therefore, the trains are transmitted for a duration:

$$T_{tg\_on} = k \cdot T_{tr} \quad (11)$$

where k is an integer greater than one, equal to the number of trains Tr in a group of trains TG, and the entire duration of a group of trains $T_{tg}$ is

$$T_{tg} = T_{tg\_on} + T_{tg\_off} = k \cdot T_{tr} + T_{tg\_off} \quad (12)$$

For example, in the currently preferred embodiment, the group of trains TG comprises four trains $Tr_1$, $Tr_2$, $Tr_3$ and $Tr_4$, i.e. k = 4, and the length of the pause $T_{tg\_off}$ is 1 s. Therefore, in the embodiment considered, the duration $T_{tg\_on}$ of a group of trains TG would be 9 s.

In this case, too, it may be envisaged that the polarity of the trains Tr included within a group of trains TG is reversed at the end of a group of trains TG, or rather at the end of a set time, for example 2 mins, or 120s. In fact, the inventors observed that reversal in the packets as shown in Figure 5 only slightly improves the result, and that efficiency increases considerably only with polarity reversal in the trains and groups of trains. Therefore, in the currently preferred embodiment, the packets within a train Tr have the same polarity.

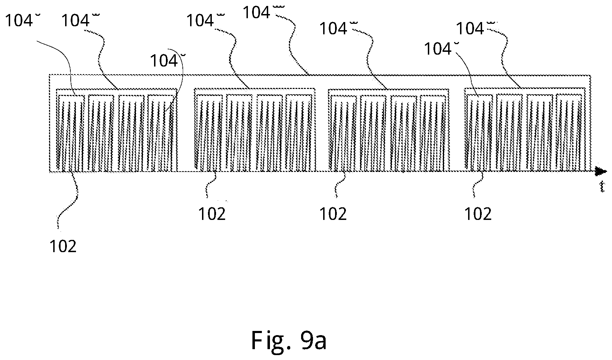

For example, Figure 9 shows a particularly preferred embodiment showing details of pulses P11, P12, P13 and P14 contained in the train $Tr1_1$. Pulses P21... etc.. contained in the train $Tr1_2$ are also shown, as well as pulses P31... etc.. contained in the train $Tr1_3$, and pulses P41... etc.. contained in the train $Tr1_4$

Pulse trains $Tr1_1$, $Tr1_2$, $Tr1_3$ and $Tr1_4$ form a first group of trains $TG_1$.

The subsequent groups of trains, not shown here, for example TG2, TG3 and TG4 can have the same polarity as the first group of trains $TG_1$, or reversed polarity between subsequent groups of trains TG2, TG3 and TG4.

Therefore the polarity of the second group of trains $TG_2$ is reversed, for instance, for the four subsequent trains

of packets $Tr2_1$, $Tr2_2$, $Tr2_3$, $Tr2_4$ contained in said second group of trains TG2; (to simplify matters, said trains of packets are not shown here but can be clearly inferred from the description of the preceding figures).

According to a particularly preferred example, the polarity of the groups of trains is reversed every 120s. Consequently, the first four trains of packets $Tr1_1$, $Tr1_2$, $Tr1_3$ and $Tr1_4$ have the same waveform and the second four trains of packets $Tr2_1$, $Tr2_2$, $Tr2_3$, $Tr2_4$ have the same waveform as the first group of packets, but with reversed polarity.

Note that within a given train of packets P11, P12, P13 and P14, the carrier signal frequencies fp11, fp12, fp13 and fp14 are the same, as is the case for fp21, fp22 and so on.

In various embodiments, the groups of trains TG are repeated for a certain duration that corresponds to the duration of treatment. For example, for typical applications, treatment duration is between 10 and 40 minutes, preferably 20 minutes.

It should be noted that the "resonance" frequencies are due to the repetition frequency of the packets, to the repetition frequency of the trains Tr and to the repetition frequency of the groups of trains $TG_i$, where, as specified previously, each group of trains $TG_i$ comprises a plurality of trains of packets transmitted for a duration $T_{tg\_on}$ followed by a pause $T_{tg\_off}$.

The length of the pauses ($T_{tg\_off}$) between groups of trains is preferably between 0.1 and 5 s inclusive.

See Table 1 below, as an example, in which burst frequency is used to indicate the frequency of the carrier signal fp:

TABLE 1

| Program | burst frequency | packet frequency | train frequency | Pause groups of trains |
|---|---|---|---|---|
| Base | Hz | Hz | Hz | Seconds |
| 1 | Varies according to depth | 2.89 | 0.4 | 0.5 |
| 2 | Idem | 3.98 | 0.5 | 0.5 |
| 3 | Idem | 6.71 | 0.9 | 0.5 |
| 4 | Idem | 8.71 | 1.1 | 0.5 |
| 5 | Idem | 10.73 | 1.4 | 0.5 |
| 6 | Idem | 13.07 | 1.6 | 0.5 |
| 7 | Idem | 15.87 | 2.1 | 0.5 |
| 8 | Idem | 21.85 | 2.8 | 0.5 |
| 9 | Idem | 16.03 | 2.8 | 0.5 |

More specifically, the device described is configured to generate a driving signal having a first depth frequency of the carrier signal 102 correlated with the depth of an organ to be treated and at least a second depth frequency correlated with the thickness of said organ.

Figure 9a, on the other hand, defines the pulses, packets, trains and trains of trains in terms of signal, so as to define the actions performed by the innovative device 1. Specifically, carrier signal 201 defines the pulses, the aforesaid modulating signal 104 is further modulated as appropriate: in particular, a signal 104' modulates and groups the pulses into packets P, a modulating signal 104" modulates and groups the packets P into trains Tr, which can have the same or different pulse frequencies within a group of trains Tg and the signal 104'''' modulates and groups the groups of trains into groups of groups of trains and can reverse the polarity. The modulating signal shall always be labelled 104 and shall accordingly take on the characteristics described. The captions 104', 104", 104''' and 104'''' shall only be used to define the various packets of signals (and/or of trains, etc.) in schematic form, as described in the figure. Note that said signals are emitted at the same time to cover, as mentioned, the emission of the full spectrum of resonance frequencies of the area to be treated, those frequencies being univocally defined by the device in accordance with specific tables taken from scientific data, to act exclusively on said area to be treated.

The inventors observed that with this wave generator - in itself generic and programmable - various treatment programs can be created.

For example, the user can select the most suitable treatment program based on the organ to be treated.

First of all, the depth of action is selected, by choosing from the depths of action from A to Z set out in the table described above with reference to Figure 6.

In a second step, the thickness of the organ to be treated is selected, where the thickness that can be selected ranges from a minimum of 0.5 cm to a maximum of 3 centimetres.

First Example.

Let us suppose we want to treat a muscle at a depth of 2 cm, for a thickness of 3 cm. Consequently, the initial depth corresponds to the train Tr1=G.

The final depth corresponds to the train Tr4=N. The choice of resonance frequency varies according to the type of tissue to be treated.

Furthermore, the software of the device is configured to complete the intermediate trains with the intermediate depths, i.e. (Tr2=J) + (Tr3= L).

The final train of packets will therefore be TG=(G+J+L+N).

The packets forming a train always have the same carrier frequency fp.

Second Example.

Let us suppose we want to treat a bone at a depth of 5 cm for a thickness of 1 cm. Consequently, the initial depth corresponds to the train N. The final depth corresponds to the train P.

The software controlling the device is configured to complete the intermediate trains with the intermediate depths, i.e. O.

However, given that there are 4 trains, in that case the software will always double the deepest frequency.

The final train will therefore be N+O+P+P.

Essentially, the pulses IM are grouped into at least four packets p11-p12-p13-p14 with the same pulse frequency ($f_{P11}$-$f_p$12-$f_p$13-$f_p$14) to form a train TR1 where the repetition frequency of the packets is correlated with the area to be treated and in this case corresponds to the highest and/or maximum frequency, which is between 2Hz and 25.9 Hz inclusive (Table 1), while the second train TR2 has the same packet repetition frequency but the pulse frequency for all the packets of TR2 can be equal to tr1 or different and corresponding to the second depth frequency, and so on for T3 and TR4.

In general, the trains are grouped into groups of at least four trains, where the repetition frequency of the trains corresponds to the second resonance frequency, which is between 0.4 and 2.8 Hz inclusive. The repetition frequency of the groups of trains occurs with a variable pause which in this case lasts 0.5 seconds. Each group of trains therefore comprises a plurality of trains of packets followed by a pause, where the length of the pause is correlated to the precise area to be treated.

The length of the pauses between groups of trains is preferably between 0.1 and 5 s inclusive.

Figure 10 shows a handpiece 200 for a device for resonance intracellular transport, according to an aspect of the invention, where said handpiece is provided with a cover 230 and a bottom part 240 as well as with a solenoid 280.

Moreover, the first electrode 20 of the device comprises an ultrasound acoustic generator 210, wherein said acoustic generator 210 is driven by means said driving signal and supported by the handpiece 200 itself.

The ultrasounds can be generated by means of a piezoelectric transducer with the following characteristics:

- Oscillation frequency between 20 and 40 kHz, preferably 20 - 25 kHz.
- Power supply voltage 12 - 24 V
- Maximum current supply 400 - 200 mA
- Power 2.5 - 3.5 W/cm$^2$

The first electrode 20 of the device may additionally comprise a magnetic transducer, wherein said magnetic transducer is driven by means of said driving signal.

By magnetic transducer, we mean a solenoid 280 placed inside the handpiece 200, with the following characteristics.

- Diameter of the cable 0.35 mm Self-sealing
- Solenoid diameter 8 cm
- Solenoid thickness 3 mm
- Coil 50
- Resistance 25 Ohm

Said solenoid 280 is driven with the same frequencies emitted for resonance.

- Power supply voltage, for example 5 V
- Current supply, for example max. 100 mA

Solenoid 280 is ring-shaped and built into the handpiece 200, generating a magnetic field all around in a coherent way.

The handpiece 200 comprises an electrification chamber 205 entirely made of a metal material to contain an active principle to be administered.

The active principle contained in the electrification (or ionisation) chamber 205 of handpiece 200 is dissolved in agarose-based gel as well as potentially in another carrier, too.

In this design, a saccharine glycoside gel is used, unlike in the case of classic transdermal transport solutions, where the gel is required to be saline.

Said solution is crucial for transporting substances for cosmetic and pharmacological use, or in general chemical substances, taking advantage of the fact that the cells prefer feeding on sugar, so in the days following application, the treated cells continue feeding, thus obtaining a long-lasting result.

For non-ionisable or heavy substances, on the other hand, (such as chemo) a further agarose-based carrier is added to break down the barrier of the stratum corneum to allow those substances to be transported.

Furthermore, the handpiece 200 comprises a roller 220 provided with knurling or grooves suitable to create microchannels in the stratum corneum of the epidermis.

The microchannels enable the gel to come into direct contact with the dermis by passing through the stratum corneum, thus cancelling out any resistance.

The handpiece 200 also comprises a dispenser 250 provided with a plane surface from which the roller 220 protrudes.

The plane surface of the dispenser 250 has an area of at least 5 cm$^2$.

An advantage of this vast plane surface is that it also makes it possible to act in the corner of the handpiece, thus increasing the area of effectiveness of the handpiece.

It should be noted that, thanks to the invention, part of the product is metabolised immediately thanks to the opening of the cell receptors as a result of the effect of resonance, while the other part of the product is left deposited in the mesenchyme as a nutritional reserve, for which reason the action will continue to be effective even up to 72 hours after.

Specifically, it is also possible to summarise the combined action of the device and handpiece as follows, by describing a working method:

- dissolving of substances to be transported in an aqueous gel, specifically, agarose gel;
- dispensing of the gel using the handpiece;
- transformation by a magnetic field produced by said solenoid to make the water clusters forming the agarose gel coherent with the currents delivered for electrophoresis and with the resonance frequencies of a tissue to be treated;

- gel deposition by gravity in the electromagnetic chamber;
- ionisation of the gel in said chamber;
- uptake of the gel by the roller, which turns and deposits it in the dermis;
- generation of mechanical action (by means of an ultrasound transducer present on the handpiece and driven by the device) to break the lipid bond of the corneocytes, thus preparing and promoting the creation of microchannels (micro tunneling) produced by the knurling and microneedles (size 2/3 micron) present on the dispenser roller;
- transport (through resonance modulated currents by means of pulses/packets/trains/groups of trains/groups of groups of trains, according to said layout) of the compound molecules present in the agarose gel saturated with active principles linked molecularly as explained previously.

Thanks to these modulations, transport occurs via the water channel and not the ion channel. The method involves the sending of currents with multiple cellular resonance frequencies at the same time.

We wish to emphasise that the known art does not provide for specific repetition and modulation frequencies and is also different in the composition of packets, where at least one of the packets within a train must have a different pulse frequency and the frequency of the packets and trains, just like their duration, is not related in a targeted way.

This method, associated with the direct use of the innovative device and handpiece, sends pulses with frequency correlated with depth (see table) (Fr = frequency; pr = depth formula Fr=(2000-180)*Pr).

- The pulses are grouped into packets, with a minimum of 4 per train, with a repetition frequency correlated to the area to be treated (table 1 column 3)
- Each train is composed of 4 packets with the same pulse frequency and therefore the same depth of transfer.
- The trains are grouped into groups of trains, with a minimum of 4 per group of trains, and the trains repeat with a frequency correlated to the area to be treated (table 1 column 2)

Uniform transfer through the thickness is obtained thanks to the increased duration of the train with the same transfer depth frequency (pulse).

- transfer of the oppositely charged compound molecules by reversing the polarity of the currents by means of the signal generator.

We wish to emphasise that this method advantageously makes it possible to transfer 60 ml of agarose gel saturated in active principles with a weight of 75g into the selected tissue in 15 minutes without dispersion in circulation and exclusively localised in the selected tissue. As noted, in the known art, 20 ml of saline gel not saturated with active principles is transferred in 1 hour and in the best case in 45 minutes, by the transdermal route, therefore making it also systemic, with dispersion in circulation.

To conclude, this description demonstrates the use of resonance, which exploits cellular communication by means of electromagnetic waves, as informational energy that enters electromagnetic resonance with the tissues and cells, which, through the DNA and the membranes, are electromagnetic emitters. Being open systems, or rather conditioned by environmental and endogenous (mind-body) stimuli, living beings can easily depart from biological vibrational harmony. In such cases, it becomes necessary to provide tested frequencies for the individual systems, or rather frequencies used and tested for treatments as described above.

**Bibliography for cellular resonance/electromagnetic fields**

1. A.R. Liboff "Cyclotron resonance in membrane transport" in A. Chiabrera et al. (Eds.) Interactions between electromagnetic fields and cells, pp. 281-296, Plenum Press, 1985

2. C.F. Blackman et al. "A role for the magnetic field in the radiation-induced efflux of calcium ions from brain tissue in vitro", Bioelectromagnetics, 6, pp. 327-337, 1985

3. Liboff A.R., Smith S.D., McLeod B.R., "Experimental evidence for ion cyclotron resonance mediation of membrane transport" in M.Blank, E.Findl (eds.) Mechanistic approaches to interactions of electromagnetic fields with living systems, New York and London: Plenum Press, pp. 109-132,1987

4. Smith S.D., McLeod B.R., Liboff A.R., Cooksey K.E., "Calcium cyclotron resonance and diatom mobility" Bioelectromagnetics, 8, 1987

5. Warnke, U. "Some primal mechanisms concerning the effects of pulsatine magnetic fields in the extremely low frequency (ELF) range on human beings." Electromagnetic Bio-information ed. F.A. Popp, ed. Munchen: Urban e Schwarzenberg, 1990

6. Grundler W., Kaiser F., Keilmann F., Walleczek J., "Mechanisms of electromagnetic interaction with cellular systems" Max Planck Institut, Stuttgart, 1991

7. F.A. Popp, Recent advances in biophoton research and its applications, World Scientific, 1992.

8. Warnke U. "Survey of some working mechanisms of pulsating electromagnetic fields (PEMF) Bioelectrochemistry and bioenergetics 27, 1992

9. Blackman C.F., Blanchard J.P., Benane S.G., House D.E., "Empirical test of an ion parametric resonance model for magnetic field interactions with PC-12 cells", Bioelectromagnetics 15:239-260, 1994

10. Blanchard J.P., Blackman C.F., "Clarification and application of an ion parametric resonance model for magnetic field interactions with biological systems", Bioelectromagnetics 15:217-238, 199

11. G. Preparata «QUED coherence in matter", World Scientific, 1995

12. R. Arani, I. Bono, E. Del Giudice, G. Preparata in E. Sassaroli et al (Eds.) "QED coherence and the thermodynamics of Water" Intern. Journal of modern Physics B9, pp.1813-1841, 1995

13. Jermann I., Berden M., Ruzic R. "Biological influence of ultraweak supposedly EM radiation from organism mediated through water" Electro- and magnetobiology, 15(3),229-44, 1996

14. Blank M., Goodman R. "Do electromagnetic fields interact directly with DNA?" Bioelectromagnetics, 18:111 5, 1997

15. Novikov, V. Karnaukov A. "Mechanism of action of weak electromagnetic Field on ionic currents in aqueous solutions of amino acids" Bioelectromagnetics, pp.18-25 , 1997

16. M.N. Zhadin et al. "Combined action of static and alternating magnetic fields on ionic current in aqueous glutamic acid solution", Bioelectromagnetics, 19, pp.. 41-45, 1998

17. E. Del Giudice, Preparata in E. Sassaroli et al. "Macroscopic Quantum Coherence", pp. 108-129 World Scientific, 1998

18. E. Del Giudice, G. Preparata, M. Fleischmann "QED coherence and electrolyte solutions" Journal of Electroanalytical Chemistry, 2000

19. M. Covelli ATTI XVI SNAMID National Conference, February 2003:"Onde elettromagnetiche e informazione biofisica. Applicazioni della biorisonanza nelle malattie osteo artro degenerative (Sistema Vital Body System)" [Electromagnetic waves and biophysical information. Applications of bioresonance in degenerative bone and joint diseases (Vital Body System)]

20. U. Greco, M.L. Roseghini, Department of Human Physiology and Pharmacology "Vittorio Espamer" of The Sapienza University of Rome, "I fenomeni di biorisonanza per un nuovo modello integrativo della diagnostica e terapia" [Bioresonance phenomena for a new integrative diagnostics and treatment model]

21. A. Laffranchi, XVII SNAMID National Congress, 20-22 FEBRUARY 2004, Milan "Il recupero delle lesioni sub-acute e croniche conseguenti alla radioterapia nel malato oncologico: 11 anni di esperienza presso l'Istituto Nazionale per lo Studio e la Cura dei Tumori di Milano" [The recovery of sub-acute and chronic lesions resulting from radiotherapy in the cancer patient: 11 years of experience at the Milan-based National Institute for the Study and Treatment of Tumours]

22. Luc Montagnier, Il DNA tra fisica e biologia onde elettromagnetiche dal DNA e acqua [DNA between physics and biology electromagnetic waves from DNA and water] Montagnier L., Aïssa J., Ferris S., Montagnier J.-L., Lavallée C. "Electromagnetic Signals Are Produced by Aqueous Nanostructures Derived from Bacterial DNA Sequences". Interdiscip Sci Comput Life Sci, 2009; 1: 81-90.

23. Montagnier L., Aissa J., Lavallée C., Mbamy M., Varon J., Chenal H. "Electromagnetic detection of HIV DNA in the blood of AIDS patients treated by antiretroviral therapy". Interdiscip Sci Comput Life Sci, 2009; 1: 245-253.

24. Del Giudice E., Tedeschi A. "Water and the autocatalysis in living matter", 2009; Electromagnetic Biology and Medicine, 28, 46.

25. Nesterov. PHYSICAL BASICS OF INFORMATIONAL INTERACTION Dedicated to 85th anniversary of the Academician Svyatoslav Pavlovich Nesterov

26. Avramenko R.F., Nikolaeva V.I., Pushkin V.N. To the matter of informational interaction of isolated systems without power transfer. // "Matters of psychohygiene, psychophysiology and labour sociology in coal industry and psychoenergetics" Moscow, NTGO, 1980, pp. 341-357

27. Akimov A. E. Quantum non-locality and torsion fields. (together with

28. Blinkov I.L. Structural-resonance (contact) and electromagnetic (contactless) simulation Theoretical and clinical aspects of bioresonance multiresonance therapy: theses of the 2n International Conference. Moscow

29. Gariaev P.P., "Wave genetic code". Moscow, Izdatcenter, 1997.

30. Gariaev P.P., "Genetic structures as source and receiver of holographic information". Together with I.V. Prangishvili, G.G. Tertinshny and others. //Sensors and systems, 2000. No. 2(11).p2-8.

31. Gotovsky Y.V., Mhitaryan K.N. Structural conception of disease and a role of external control loop in its treatment. // Theoretical and clinical aspects of bioresonance and multiresonance therapy: Theses of the 2nd International Conference. Moscow, Imedis, 1996. P. 79_94.

32. Davydov A.S. Solitons in bioenergetics. Kiev, Nauk, Dumka, 1986. Devyatkov N.D., Golant M.B., Betsky O. V. "Millimeter waves and their role in processes of vital activity". Moscow, Radio and communication

33. Smith S. "Electromagnetic bio-information and water". // Biophysical medi_cine bulletin, 1994. No 1. pp. 3-13.

34. Fralich G. "Coherent excitations in biological systems". // Biophysics, 1977. Vol. 22. No. 4. P. 743_744Henzel

35. Cherkasov A.V. Magneto-optical influence on water-dependant structures of living organism. // Biophysical medicine bulletin, 1994. No.1. P. 49_52.

36. Chirkova E.N. Wave nature of regulation of genetic activity: living cell as photon computer. // Russian thought, 1992. No.2. P. 29_41.

37. Chuguevsky A.V., Fedorenko N.E. Electromagnetic solitons. Moscow, 1980, 80 pages. VINITI No.8280.

38. Bertalanffy L. "Das biologische" Weltbild. Bd I.Bem, 1949.

39. Frohlich H. "Biological coherence and response to external stimuli". Berlin, Heiderberg, Sprinder, 1988

40. Popp F.A. "Electromagnetic Bio-information" / Ed. F.A. Popp et al. Urban und Schwarzenberg Munchen - Baltimore, 1979. Smith C.W. "Electromagnetic phenomena in living biomedical systems" //Proc. 6_th. Am. Conf._1984._P. 176_180. Smith C.W., Choy R., Monroe J.A. "Water, Friend or Foe?" // Laboratory Practice. 1985._Vol. 34._P. 29_34.

41. Lagendijk A., van Tiggelen B.A., "Resonant Multiple Scattering of Light", Physics Reports, v.270, pp. 143-216, 1996.

## Claims

1. A device (1) for intracellular transport of an active principle dissolved in a gel, wherein said device comprises two electrodes (20, 30), wherein a first of said electrodes (20) is configured to contain the active principle to be administered, the device further comprising a wave generator (10) configured to generate a driving signal to be sent to said electrodes (20, 30), wherein said driving signal includes a plurality of packets (P) grouped into trains of packets (Tr) and into groups of trains (TG), wherein each packet (P) consists of a unidirectional signal resulting from the combination of a modulating signal (104) and a carrier signal (102), wherein each train of packets (Tr) consists of a series of packets (P), in which each group of trains (TG) comprises a series of trains of packets (Tr), and wherein said wave generator (10) is configured to at least reverse the polarity of said trains of packets (Tr); the device being configured to generate said carrier signals (102), wherein the carrier signals of each train of packets (Tr) have a frequency $f_p$ dependent upon a penetration depth p, the frequency $f_p$ being calculated according to the following formula:

$$f_{p=} \left[ 2000 - \frac{180*p}{cm} \right] Hz,$$

**characterized in that** the device further comprises a handpiece (200, said first electrode (20) is connected to the handpiece (200) and comprises a magnetic transducer, wherein said magnetic transducer is driven by means of said driving signal, said magnetic transducer comprising a solenoid (280) having a ring shape and being built into said handpiece (200), said device being configured to generate at least a further driving signal generated by said wave generator (10) to drive the solenoid and **in that** said device (1) is configured to generate at least further modulating signals (104) to build waveforms comprising packets (P) and trains of packets (Tr) and groups of trains of packets (Tg) and groups of groups of trains, said device being configured to generate a first train of packets (Tr1) having a first carrier signal frequency (fp11), a second train of packets (Tr4) having a second carrier signal frequency (fp14), said second carrier signal frequency (fp4) being lower than the first carrier signal frequency (fp1), and to generate intermediate train of packets (Tr2, Tr3) having carrier signal frequencies (fp12,fp13) that are intermediate between said first carrier signal frequency (fp11) and said second carrier signal frequency (fp14), said frequencies being calculated to act exclusively at the desired penetration depth.

2. The device according to claim 1, wherein at least one modulating signal (104') groups and modulates that pulse into at least four packets (P) having the same pulse frequency.

3. The device according to the preceding claims, wherein at least one modulating signal (104") groups and modulates those packets (P) into trains of packets (Tr) which may have equal or different pulse frequencies within a group of trains.

4. The device according to the preceding claims, wherein at least one modulating signal (104''') groups and modulates the trains (Tr) into groups of trains (Tg), and the modulating signal (104"") modulates and groups the groups of trains into groups of groups of trains and reverses the polarity of the packets with respect to the polarity of the packets in the previous group of trains.

5. The device according to the preceding claims, wherein each group of trains (TG) comprises a plurality of trains of packets (Tr) followed by a pause (Ttg_off).

6. The device according to claim 1, wherein said first electrode (20) is connected to a handpiece (200) that comprises an ultrasound acoustic generator (210), wherein said ultrasound acoustic generator (210) is driven by means of said driving signal.

7. The device according to claim 6, wherein the ultrasound acoustic generator (210) operates at a frequency of between 20 and 40 kHz inclusive.

8. The device according to claim 1, wherein the handpiece (200) comprises an electrification chamber (205) entirely made of a metal material to contain an active principle to be administered.

9. The device according to claim 8, wherein the active principle contained in the electrification chamber (205) of the handpiece (200) is dissolved in agarose-based gel.

10. The device according to claim 7, wherein the handpiece (200) comprises a roller (220) having an external surface provided with knurling suitable to create microchannels in the stratum corneum of the epidermis.

11. The device according to claim 9, wherein the handpiece (200) comprises a dispenser (250) provided with a plane surface from which said roller (220) protrudes, wherein said plane surface of the dispenser (250) has an area no smaller than 5 cm$^2$.

**Patentansprüche**

1.  Vorrichtung (1) zum intrazellulären Transport eines in einem Gel gelösten Wirkstoffs, wobei die Vorrichtung zwei Elektroden (20, 30) umfasst, wobei eine erste der Elektroden (20) so konfiguriert ist, dass sie den zu verabreichenden Wirkstoff enthält , die Vorrichtung, die ferner einen Wellengenerator (10) umfasst, der konfiguriert ist, um ein Ansteuersignal zu erzeugen, das an die Elektroden (20, 30) gesendet werden soll, wobei das Ansteuersignal mehrere Pakete (P) enthält, gruppiert in Züge von Paketen (Tr) und in Gruppen von Zügen (TG), wobei jedes Paket (P) aus einem unidirektionalen Signal besteht, das sich aus der Kombination eines modulierenden Signals (104) und eines Trägersignals (102) ergibt, wobei jeder Paketzug (Tr) aus einer Reihe von Paketen (P) besteht, wobei jede Gruppe von Zügen (TG) eine Reihe von Paketen (Tr) umfasst und wobei der Wellengenerator (10) so konfiguriert ist, dass er zumindest die Polarität der Paketzüge (Tr) umkehrt; die Vorrichtung ist konfiguriert, um die Trägersignale (102) zu erzeugen, wobei die Trägersignale jedes Paketzugs (Tr) eine Frequenz fp aufweisen, die von einer Eindringtiefe p abhängt, wobei die Frequenz fp gemäß der folgenden Formel berechnet wird:

$$f_p \left[ 2000 - \frac{180*p}{cm} \right] Hz$$

    **dadurch gekennzeichnet, dass** die Vorrichtung ferner ein Handstück (200) umfasst, die erste Elektrode (20) ist mit dem Handstück (200) verbunden und umfasst einen Magnetwandler, wobei der Magnetwandler mittels des Ansteuersignals angesteuert wird, wobei der Magnetwandler einen Magneten (280) mit einer Ringform umfasst und in das Handstück (200) eingebaut ist, wobei die Vorrichtung konfiguriert ist, um mindestens ein weiteres Ansteuersignal zu erzeugen, das von dem Wellengenerator (10) erzeugt wird, um den Magneten anzutreiben, und in dieser ist die Vorrichtung (1) konfiguriert, um mindestens weitere Modulationssignale (104) zu erzeugen, um Wellenformen aufzubauen, die Pakete (P) und Gruppen von Gruppen von Zügen umfassen, wobei die Vorrichtung konfiguriert ist, um einen ersten Zug von Paketen (Tr1) mit einer ersten Trägersignalfrequenz (fpll), einen zweiten Zug von Paketen (Tr4) mit einer zweiten Trägersignalfrequenz (fp14) zu erzeugen, die zweite Trägersignalfrequenz (fp4) ist niedriger als die erste Trägersignalfrequenz (fp1) um einen Zwischenzug von Paketen (Tr2, Tr3) mit Trägersignalfrequenzen (fp12, fp13) zu erzeugen, die zwischen der ersten Trägersignalfrequenz (fp11) und der zweiten Trägersignalfrequenz (fp14) liegen, wobei die Frequenzen so berechnet werden, dass sie ausschließlich bei der gewünschten Eindringtiefe wirken.

2.  Die Vorrichtung nach Anspruch 1, wobei mindestens ein Modulationssignal (104') diesen Impuls gruppiert und in mindestens vier Pakete (P) mit derselben Impulsfrequenz moduliert

3.  Die Vorrichtung nach den vorhergehenden Ansprüchen, wobei mindestens ein modulierendes Singular (104") diese Pakete (P) in Züge von Paketen (Tr) gruppiert und moduliert, die gleiche oder unterschiedliche Impulsfrequenzen innerhalb einer Gruppe von Zügen aufweisen können.

4.  Die Vorrichtung nach den vorhergehenden Ansprüchen, wobei mindestens ein Modulationssignal (104"') die Züge (Tr) in Gruppen von Zügen (Tg) gruppiert und moduliert und das Modulatinsignal (104"") das Gruppen von Zügen in Gruppen von Gruppen von Zügen moduliert und gruppiert und die Polarität der Pakete in Bezug auf die Polarität der Pakete in der vorherigen Gruppe von Zügen umkehrt.

5.  Die Vorrichtung nach den vorhergehenden Ansprüchen, wobei jede Gruppe von Zügen (TG) mehrere Züge von Paketen (Tr) umfasst, gefolgt von einer Pause (Ttg_off).

6.  Die Vorrichtung nach Anspruch 1, wobei die erste Elektrode (20) mit einem Handstück (200) verbunden ist, das einen akustischen Ultraschall-Akustikgenerator (210) umfasst, wobei der akustische Ultraschall-Akustikgenerator (210) mittels des Ansteuersignals angesteuert wird.

7.  Die Vorrichtung nach Anspruch 6, wobei der Ultraschall-Akustikgenerator (210) bei einer Frequenz von 20 bis einschließlich 40 kHz arbeitet.

**8.** Die Vorrichtung nach Anspruch 1, wobei das Handstück (200) eine Elektrifizierungskammer (205) umfasst, die vollständig aus einem Metallmaterial besteht, um einen zu verabreichenden Wirkstoff zu enthalten.

**9.** Die Vorrichtung nach Anspruch 8, wobei der in der Elektrifizierungskammer (205) des Handstücks (200) enthaltene Wirkstoff in Gel auf Agarosebasis gelöst ist.

**10.** Die Vorrichtung nach Anspruch 7, wobei das Handstück (200) eine Walze (220) mit einer Außenfläche umfasst, die mit Rändelung geeigneter Mikrokanäle im Stratum Corneum der Epidermis versehen ist.

**11.** Vorrichtung nach Anspruch 9, wobei das Handstück (200) einen Spender (250) umfasst, der mit einer ebenen Oberfläche versehen ist, aus der die Walze (220) herausragt, wobei die ebene Oberfläche des Spenders (250) eine Fläche von nicht weniger als 5 cm$^2$ aufweist.

**Revendications**

**1.** Un dispositif (1) pour le transport intracellulaire d'un principe actif dissous dans un gel, dans lequel ledit dispositif comprend deux électrodes (20, 30), où une première desdites électrodes (20) est configurée pour contenir le principe actif à administrer, le dispositif comprenant en outre un générateur d'ondes (10) configuré pour générer un signal de commande à envoyer auxdites électrodes (20, 30), dans lequel ledit signal de commande comprend une pluralité de paquets (P) regroupés en trains de paquets (Tr) et en groupes de trains (TG), où chaque paquet (P) est constitué d'un signal unidirectionnel résultant de la combinaison d'un signal de modulation (104) et d'un signal porteur (102), dans lequel chaque train de paquets (Tr) est constitué d'une série de paquets (P), dans lequel chaque groupe de trains (TG) comprend une série de trains de paquets (Tr), et dans lequel ledit générateur d'ondes (10) est configuré pour au moins inverser la polarité desdits trains de paquets (Tr) ; le dispositif étant configuré pour générer lesdits signaux porteurs (102), où les signaux porteurs de chaque train de paquets (Tr) ont une fréquence $f_p$ dépendant d'une profondeur de pénétration p, la fréquence $f_p$ étant calculée selon la formule suivante:

$$f_p \left[ 2000 - \frac{180 * p}{cm} \right] Hz$$

**caractérisée en ce que** le dispositif comprend en outre une pièce à main (200), ladite première électrode (20) est connectée à la pièce à main (200) et comprend un transducteur magnétique, dans lequel ledit transducteur magnétique est entraîné au moyen dudit signal de commande, ledit transducteur magnétique comprenant un solénoïde (280) ayant une forme annulaire et étant construit dans ladite pièce à main (200), ledit dispositif étant configuré pour générer au moins un autre signal de commande généré par ledit générateur d'ondes (10) pour entraîner le solénoïde et **en ce que** ledit dispositif (1) est configuré pour générer au moins d'autres signaux de modulation (104) pour construire des formes d'onde comprenant des paquets (P) et des trains de paquets (Tr) et des groupes de trains de paquets (Tg) et des groupes de groupes de trains, ledit dispositif étant configuré pour générer un premier train de paquets (Tr1) ayant une première fréquence de signal porteur (fp11), un second train de paquets (Tr4) ayant une seconde fréquence de signal porteur (fp14), ladite seconde fréquence de signal porteur (fp4) étant inférieure à la première fréquence de signal porteur (fp1), et pour générer un train intermédiaire de paquets (Tr2, Tr3) ayant des fréquences de signal porteur (fp12, fp13) qui sont intermédiaires entre ladite première fréquence de signal porteur (fp11) et ladite seconde fréquence de signal porteur (fp14), lesdites fréquences étant calculées pour agir exclusivement à la profondeur de pénétration souhaitée.

**2.** Le dispositif selon la revendication 1, dans lequel au moins un signal de modulation (104') regroupe et module cette impulsion en au moins quatre paquets (P) ayant la même fréquence d'impulsion.

**3.** Le dispositif selon les revendications précédentes, dans lequel au moins un signal de modulation (104") regroupe et module ces paquets (P) en trains de paquets (Tr) qui doivent avoir des fréquences d'impulsions égales ou différentes dans un groupe de trains.

**4.** Le dispositif selon les revendications précédentes, dans lequel au moins un signal de modulation (104 " ') regroupe et module les trains (Tr) en groupes de trains (Tg), et le signal de modulation (104" ") module et regroupe les groupes de trains en groupes de groupes de trains et inverse la polarité des paquets par rapport à la polarité des paquets dans le dans le groupe de trains précédent.

**5.** Le dispositif selon les revendications précédentes, dans lequel chaque groupe de trains (TG) comprend une pluralité de trains de paquets (Tr) suivie d'une pause (Ttg_off).

**6.** Le dispositif selon la revendication 1, dans lequel ladite première électrode (20) est connectée à une pièce à main (200) qui comprend un générateur acoustique à ultrasons (210), dans lequel ledit générateur acoustique à ultrasons (210) est entraîné au moyen dudit signal de commande.

**7.** Le dispositif selon la revendication 6, dans lequel le générateur acoustique d'ultrasons (210) fonctionne à une fréquence comprise entre 20 et 40 kHz inclue.

**8.** Le dispositif selon la revendication 1, dans lequel la pièce à main (200) comprend une chambre d'électrification (205) entièrement réalisée en un matériau métallique pour contenir un principe actif à administrer.

**9.** Le dispositif selon la revendication 8, dans lequel le principe actif contenu dans la chambre d'électrification (205) de la pièce à main (200) est dissous dans un gel à base d'agarose.

**10.** Le dispositif selon la revendication 7, dans lequel la pièce à main (200) comprend un rouleau (220) ayant une surface externe pourvue d'un moletage approprié pour créer des micro-canaux dans la couche cornée de l'épiderme.

**11.** Le dispositif selon la revendication 9, dans lequel la pièce à main (200) comprend un distributeur (250) pourvu d'une surface plane à partir de laquelle ledit rouleau (220) fait saillie, dans lequel ladite surface plane du distributeur (250) a une aire non inférieure à 5 cm$^2$.

*Fig. 1*

Fig. 1a

Fig. 2

Fig. 3

**Fig. 4**

**Fig. 5**

| | p [cm] | f [Hz] |
|---|---|---|
| A | 0.1 | 1982 |
| B | 0.2 | 1954 |
| C | 0.3 | 1946 |
| D | 0.5 | 1910 |
| E | 1 | 1820 |
| F | 1.5 | 1730 |
| G | 2 | 1640 |
| H | 2.5 | 1550 |
| I | 3 | 1460 |
| J | 3.5 | 1370 |
| K | 4 | 1280 |
| L | 4.5 | 1190 |
| M | 5 | 1100 |
| N | 5.5 | 1010 |
| O | 6 | 920 |
| P | 6.5 | 830 |
| Q | 7 | 740 |
| R | 7.5 | 650 |
| S | 8 | 560 |
| T | 8.5 | 470 |
| U | 9 | 380 |
| V | 9.5 | 290 |
| W | 10 | 200 |

*Fig. 6*

*Fig. 7*

Fig. 8

FIG.9

Fig. 9a

FIG.10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT FI990141 A
- IT FI980137 A
- IT FI990055 A
- WO 0074774 A

- WO 2013190489 A
- US 20030233085 A
- US 20110098632 A
- US 20140114234 A


**Non-patent literature cited in the description**

- ATTI XVI SNAMID National Congress. **M. COVELLI.** Biological coherence and response to external stimuli. Sprinder, February 2003, 1988
- Cyclotron resonance in membrane transport. **A.R. LIBOFF et al.** Interactions between electromagnetic fields and cells. Plenum Press, 1985, 281-296
- **F. A. POPP.** Recent Advances in biophoton research and its application. *World Scientific,* 1992
- **BLANK MARTIN ; GOODMAN REBA.** Do electromagnetic fields interact directly with DNA?. *Bioelectromagnetics,* 1997, vol. 18 (111), 5
- **GARIAEV P.P.** Wave genetic code. Izdatcenter, 1997
- Genetic structures as source and receiver of holographic information. **GARIAEV P.P.** Sensors and systems. 2000, vol. 11, 2-8
- **C.F. BLACKMAN et al.** A role for the magnetic field in the radiation-induced efflux of calcium ions from brain tissue in vitro. *Bioelectromagnetics,* 1985, vol. 6, 327-337
- Experimental evidence for ion cyclotron resonance mediation of membrane transport. **LIBOFF A.R. ; SMITH S.D. ; MCLEOD B.R.** Mechanistic approaches to interactions of electromagnetic fields with living systems. Plenum Press, 1987, 109-132
- **SMITH S.D. ; MCLEOD B.R. ; LIBOFF A.R. ; COOKSEY K.E.** Calcium cyclotron resonance and diatom mobility. *Bioelectromagnetics,* 1987, vol. 8
- Some primal mechanisms concerning the effects of pulsatine magnetic fields in the extremely low frequency (ELF) range on human beings. **WARNKE, U.** Electromagnetic Bio-information. 1990
- **GRUNDLER W. ; KAISER F. ; KEILMANN F. ; WALLECZEK J.** Mechanisms of electromagnetic interaction with cellular systems. Max Planck Institut, 1991
- **F.A. POPP.** Recent advances in biophoton research and its applications. *World Scientific,* 1992

- **WARNKE U.** Survey of some working mechanisms of pulsating electromagnetic fields (PEMF). *Bioelectrochemistry and bioenergetics,* 1992, vol. 27
- **BLACKMAN C.F. ; BLANCHARD J.P ; BENANE S.G. ; HOUSE D.E.** Empirical test of an ion parametric resonance model for magnetic field interactions with PC-12 cells. *Bioelectromagnetics,* 1994, vol. 15, 239-260
- **BLANCHARD J.P ; BLACKMAN C.F.** Clarification and application of an ion parametric resonance model for magnetic field interactions with biological systems. *Bioelectromagnetics,* vol. 15, 217-238, 199
- **G. PREPARATA.** QUED coherence in matter. *World Scientific,* 1995
- QED coherence and the thermodynamics of Water. Intern. Journal of modern Physics. 1995, vol. B9, 1813-1841
- **JERMANN I. ; BERDEN M. ; RUZIC R.** Biological influence of ultraweak supposedly EM radiation from organism mediated through water. *Electro- and magnetobiology,* 1996, vol. 15 (3), 229-44
- **BLANK M. ; GOODMAN R.** Do electromagnetic fields interact directly with DNA?. *Bioelectromagnetics,* 1997, vol. 18, 111-5
- **NOVIKOV, V. ; KARNAUKOV A.** Mechanism of action of weak electromagnetic Field on ionic currents in aqueous solutions of amino acids. *Bioelectromagnetics,* 1997, 18-25
- **M.N. ZHADIN et al.** Combined action of static and alternating magnetic fields on ionic current in aqueous glutamic acid solution. *Bioelectromagnetics,* 1998, vol. 19, 41-45
- **E. DEL GIUDICE ; E. SASSAROLI et al.** Macroscopic Quantum Coherence. *World Scientific,* 1998, 108-129
- **E. DEL GIUDICE ; G. PREPARATA ; M. FLEISCHMANN.** QED coherence and electrolyte solutions. *Journal of Electroanalytical Chemistry,* 2000

- **M. COVELLI.** Onde elettromagnetiche e informazione biofisica. Applicazioni della biorisonanza nelle malattie osteo artro degenerative (Sistema Vital Body System)'' [Electromagnetic waves and biophysical information. Applications of bioresonance in degenerative bone and joint diseases (Vital Body System). *ATTI XVI SNAMID National Conference,* February 2003
- **U. GRECO ; M.L. ROSEGHINI.** I fenomeni di biorisonanza per un nuovo modello integrativo della diagnostica e terapia'' [Bioresonance phenomena for a new integrative diagnostics and treatment model. *Department of Human Physiology and Pharmacology ''Vittorio Espamer'' of The Sapienza University of Rome*
- **A. LAFFRANCHI.** Il recupero delle lesioni sub-acute e croniche conseguenti alla radioterapia nel malato oncologico: 11 anni di esperienza presso l'Istituto Nazionale per lo Studio e la Cura dei Tumori di Milano'' [The recovery of sub-acute and chronic lesions resulting from radiotherapy in the cancer patient: 11 years of experience at the Milan-based National Institute for the Study and Treatment of Tumours. *XVII SNAMID National Congress,* 20 February 2004
- **MONTAGNIER L. ; AÏSSA J. ; FERRIS S. ; MONTAGNIER J.-L. ; LAVALLÉE C.** Electromagnetic Signals Are Produced by Aqueous Nanostructures Derived from Bacterial DNA Sequences. *Interdiscip Sci Comput Life Sci,* 2009, vol. 1, 81-90
- **MONTAGNIER L. ; AISSA J. ; LAVALLÉE C. ; MBAMY M. ; VARON J. ; CHENAL H.** Electromagnetic detection of HIV DNA in the blood of AIDS patients treated by antiretroviral therapy. *Interdiscip Sci Comput Life Sci,* 2009, vol. 1, 245-253
- **DEL GIUDICE E. ; TEDESCHI A.** Water and the autocatalysis in living matter. *Electromagnetic Biology and Medicine,* 2009, vol. 28, 46
- **NESTEROV.** Dedicated to 85th anniversary of the Academician Svyatoslav Pavlovich Nesterov. *PHYSICAL BASICS OF INFORMATIONAL INTERACTION*
- **AVRAMENKO R.F. ; NIKOLAEVA V.I. ; PUSHKIN V.N.** Matters of psychohygiene, psychophysiology and labour sociology in coal industry and psychoenergetics. *To the matter of informational interaction of isolated systems without power transfer,* 1980, 341-357

- **AKIMOV A. E.** *Quantum non-locality and torsion fields*
- **BLINKOV I.L.** Structural-resonance (contact) and electromagnetic (contactless) simulation Theoretical and clinical aspects of bioresonance multiresonance therapy. *theses of the 2n International Conference*
- **GOTOVSKY Y.V. ; MHITARYAN K.N.** Structural conception of disease and a role of external control loop in its treatment. *Theoretical and clinical aspects of bioresonance and multiresonance therapy: Theses of the 2nd International Conference,* 1996, 79-94
- Millimeter waves and their role in processes of vital activity. **DAVYDOV A.S.** Solitons in bioenergetics. Radio and communication, 1986
- **SMITH S.** Electromagnetic bio-information and water. *Biophysical medi_cine bulletin,* 1994, 3-13
- **FRALICH G.** Coherent excitations in biological systems. *Biophysics,* 1977, vol. 22 (4), 743-744
- **CHERKASOV A.V.** Magneto-optical influence on water-dependant structures of living organism. *Biophysical medicine bulletin,* 1994, 49-52
- **CHIRKOVA E.N.** Wave nature of regulation of genetic activity: living cell as photon computer. *Russian thought,* 1992, 29-41
- **CHUGUEVSKY A.V. ; FEDORENKO N.E.** *Electromagnetic solitons,* 1980, vol. 80 (8280
- **BERTALANFFY L.** Das biologische. *Weltbild.,* 1949, vol. I
- **FROHLICH H.** Biological coherence and response to external stimuli. Sprinder, 1988
- Electromagnetic Bio-information. **POPP F.A. et al.** Urban und Schwarzenberg Munchen. 1979
- **SMITH C.W.** Electromagnetic phenomena in living biomedical systems. *Proc. 6_th. Am. Conf.,* 1984, 176-180
- **SMITH C.W. ; CHOY R. ; MONROE J.A.** Water, Friend or Foe?. *Laboratory Practice,* 1985, vol. 34, 29-34
- **LAGENDIJK A. ; VAN TIGGELEN B.A.** Resonant Multiple Scattering of Light. *Physics Reports,* 1996, vol. 270, 143-216